# EUROPEAN PATENT APPLICATION

(11) **EP 4 461 238 A1**
(43) Date of publication of application: **13.11.2024**
(21) Application number: 23172144.0
(22) Date of filing: 08.05.2023
(51) Int. Cl.: A61B 17/17

(54) **MEDICAL DEVICE WITH SLEEVE CLAMPING MECHANISM**

(71) Applicant: Stryker European Operations Limited, Carrigtwohill, Co. Cork T45 HX08 (IE)
(72) Inventor: SIMON, Bernd, 24107 Kiel (DE)
(74) Representative: Maiwald GmbH

(57) **Abstract**

Medical device with at least one clamping mechanism for fixing a secondary device to be connected to said medical device allowing a space-saving clamping and reliable fixation of secondary device to the medical device.

## Description

### Field of the invention

The present invention relates to a medical device with sleeve clamping mechanism, a surgical targeting system with said medical device and a method for manufacturing said medical device with an improved simple and cost-effective clamping mechanism for a sleeve in e.g., a target device.

### Background of the Invention

When implanting an intramedullary rod implant, the implant is usually secured by locking screws. For positioning of a locking screw, a targeting arm is used, which may be connected to the end of the rod implant that is inserted into the intramedullary canal of a long bone, such as the tibia or femur, and provide a targeting geometry, which allows exact positioning of drill sleeves to provide the trajectory of locking screws in various directions. In order to fix such a drill sleeve, a clamp may be used, which allows keeping the position of the drill sleeve at a certain position, so that position control may be carried out by x-ray imaging or other preparations may be carried out before continuing the implantation. The reliability of the clamp for maintaining the fixed position is crucial for the success of the surgery.

Medical devices with clamps for locking implants or instruments/tools at a targeting device or the like are known from, e.g., EP 1 346 696 A2, which describes a clamp at a targeting tool. Further, a clamp for a tool is known from EP 2 810 606 A1, which describes another type of clamp. Another type of clamp is known from US 4,920,958 and also from EP 1 698 286 A1.

### Summary of the Invention

The present invention provides a medical device with a clamp with improved clamping performance, a surgical targeting system and a method for manufacturing a medical device with a clamp according to the independent claims, whereas further embodiments are incorporated in the dependent claims.

According to an embodiment, there is provided a medical device with at least one clamping mechanism for fixing a secondary device to be connected to said medical device, wherein the medical device comprises a medical device body and a cantilever, wherein the medical device body comprises a first through-hole extending along a first axis, a third through-hole which is aligned to the first through-hole along the first axis, and a space at the medical device body between the first through-hole and the third through-hole, wherein the cantilever in a resting position extends into the space between the first through-hole and the third through-hole traverse to the first axis and has a second through-hole, wherein the second through-hole in the resting position has a maximum cross-sectional projection along a second axis being inclined to the first axis, wherein the first through-hole, the second through-hole and the third through-hole form the at least one clamping mechanism.

The term "space at" may be a space formed within the medical device body or a space being partially surrounded by the medical device body. In case it is referred to a slit in the space, this means a slit in the medical device body at a face of the medical device body, which forms at least one boundary of the space.

The term "resting position" of a cantilever is to be understood as the position where no forces act on the cantilever. It is understood that the gravity force acting on the cantilever does not move the cantilever out of the resting position. In comparison thereto, a "deflected position" is a position of the cantilever, which is achieved by a force acting on the cantilever traverse to the extension direction of the cantilever and distant from the cantilever fixing point, i.e., a torque, and which moves the cantilever out of the resting position. A "deflected position" can also be achieved by blocking the deflected cantilever from returning to its resting position, e.g., when the first, second and third through-hole have received a secondary device, like a drill sleeve. As a cantilever intrinsically is mounted on only one side, a torque will deflect the cantilever and thus will also incline the second axis along which the second through-hole extends with respect to an extension of the second axis in its resting position.

The term "traverse" means that the extension of the cantilever is not along the aligning axis of the both through-holes but intersects the aligning axis. "Traverse" may also be orthogonal.

The term "cross-sectional projection" of a through-hole along an axis means a projection of the cross-section, which is open at any depth of the through-hole along said axis. "Maximum cross-sectional projection" can be considered as a cross-section of a (not mandatorily circular) cylinder along said axis with a maximal possible cross-section, which still passes the through-hole. The same definition applies for more than one through-hole, in particular when considering an effective cross-sectional projection of all those through-holes together, which may differ from the cross-sectional projection of each of the single through-holes.

Thus, a medical device may be provided which allows for actuation of a clamping mechanism at one side of a targeting jig while being cost-effective due to simple manufacturing and assembly. The clamping mechanism may be used for fixation of secondary devices, e.g., a targeting sleeve or drill sleeve. The drill sleeves in a fixed position may be oriented toward a drill target, e.g., a bone with an already implanted implant. The first and third through-hole allow for a defined guiding of the secondary device, which can be fixed to the medical device, in particularly in case the medical device is used as a targeting device. The second through-hole is provided on a cantilever, so that deflecting the cantilever will change the inclination of an axis along which the second through-hole in the cantilever extends. In case the first, second and third through-hole have the same cross-section, e.g., but not mandatorily a circuit, the maximum cross-section of the first and third through-hole may be orthogonal to the same extending axis along which the through-holes extend, which corresponds to a drilling direction when manufacturing the respective through-hole. The maximum cross-section of the second through-hole in the cantilever in its un-deflected position is orthogonal to an extending axis of the second through-hole, which extending axis is inclined over the extending axis of the first and third through-hole. In case the cantilever is deflected, the first, second and third through-hole may be brought into alignment, so that the inclination of the first axis over the second axis is reduced and an effective cross-section of the first, second and third through-hole is enlarged, so a secondary device having a corresponding outer shape with a clearance fit so that it may pass. As soon as the cantilever is released, the second through-hole leaves the aligned position and clamps the secondary device over the first and third through-hole. This arrangement forms the at least one clamping mechanism.

According to an embodiment, the medical device comprises a further cantilever, wherein the medical device body comprises a further first through-hole extending along a further first axis, a further third through-hole which is aligned to the further first through-hole along the further first axis, and a further space at the medical device body between the further first through-hole and the further third through-hole, wherein the further cantilever in a resting position extends into the further space between the further first through-hole and the further third through-hole traverse to the further first axis and has a further second through-hole, wherein the further second through-hole in the resting position has a maximum cross-sectional projection along a further second axis being inclined to the further first axis wherein the further first through-hole, the further second through-hole and the further third through-hole form a second clamping mechanism. The spaces of different clamping mechanisms may form a joint space.

Thus, a medical device may be provided which has at least two clamping mechanisms for fixing a secondary device, e.g., a drill sleeve, at two different positions and possibly orientations. Such device also allows fixing of two secondary devices at the same time. It should be noted that it is also possible to provide further additional first, second and third through-holes, spaces and cantilevers for providing additional clamping mechanisms.

According to an embodiment, there is provided a medical device with at least one clamping mechanism for fixing a secondary device to be connected to said medical device, wherein the medical device comprises a medical device body having a longitudinal extension, and a cantilever, wherein the medical device body comprises a first through-hole extending along a first axis, and a space at the medical device body in front of the first through-hole, wherein the cantilever in a resting position extends into the space in front of the first through-hole traverse to the first axis and traverse to the longitudinal extension of the medical device body and has a second through-hole, wherein the second through-hole in the resting position has a maximum cross-sectional projection along a second axis being inclined to the first axis, wherein the first through-hole and the second through-hole form the at least one clamping mechanism.

The term "longitudinal extension" means an extension of the medical device body from a location where the first through-hole is located and a further inventory of the medical device, which may be a coupling interface for an implant or tool to which the secondary device is oriented upon clamping. This may correspond to the extension along the largest dimension of the medical device. This may also include an extension along a bended path or an extension along a branched path. The longitudinal extension of the body may be understood as following the main material body structure.

Thus, it is possible to provide a clamping mechanism, which has a very small space consumption with respect to the longitudinal axis of the medical device. As the cantilever extends traverse to the longitudinal direction of the medical device, the body of the medical device may receive other inventory or structures close to the clamping mechanism, as the cantilever does not extend along the longitudinal extension of the body of the medical device. This may also include further clamping mechanisms.

According to an embodiment, the medical device comprises a further cantilever, wherein the medical device body comprises a further first through-hole extending along a further first axis, and a further space at the medical device body in front of the further first through-hole, wherein the further cantilever in a resting position extends into the further space in front of the further first through-hole traverse to the further first axis and traverse to the longitudinal extension of the medical device body and has a further second through-hole, wherein the further second through-hole in the resting position has a maximum cross-sectional projection along a further second axis being inclined to the further first axis, wherein the further first through-hole and the further second through-hole form a second clamping mechanism.

Thus, a medical device may be provided which has at least two clamping mechanisms for fixing a secondary device, e.g., a drill sleeve, at two different positions and possibly orientations. Such device also allows fixing of two secondary devices at the same time. It should be noted that it is also possible to provide further additional first and second through-holes, spaces and cantilevers for providing additional clamping mechanisms. The spaces of different clamping mechanisms may form a joint space. In particular, the extension of the cantilever traverse to the longitudinal extension of the medical device body allows close positioning of the two or more clamping mechanisms on the medical device, in particular as the cantilever does not extend along the longitudinal direction where other clamping mechanism or other inventories are positioned.

According to an embodiment, a minimum lateral distance d between the first through-hole and the further first through-hole is less than three times of the minimum diameter of the first through-hole, in particular less than two times of the minimum diameter of the first through-hole, in particular less than the minimum diameter of the first through-hole, in particular less than half of the minimum diameter of the first through-hole.

The term "minimum lateral distance" means the minimum distance between lateral side walls of adjacent first through-holes. In case the through-holes are parallel boreholes, the minimum distance follows the shortest way from one borehole to the other borehole. The shortest straight way usually is orthogonal to both surfaces (e.g., cylinder barrel face) of the adjacent boreholes. This also applies for the case where the boreholes are not parallel.

The term "minimum diameter of the first through-hole" means the diameter of a maximum circular cylinder just passing the through-hole. For a circular cylindrical through-hole, this is the diameter of the through-hole.

Thus, it is possible to provide two clamping mechanisms adjacent and close to each other. This allows providing a large number of clamping mechanisms along a longitudinal extension of a medical device body. Further, this allows the provision of two clamping mechanisms close to each other if this is required for an implant to be implanted. The medical device body does not need to be re-positioned in case, e.g., two drilling holes have to be made close to each other, as two drill sleeves can be positioned and fixed close to each other at the same time.

According to an embodiment, the medical device body comprises at least one of a third and further third through-hole which is aligned to the respective first/further first through-hole along the respective first/further first axis, wherein the respective space/further space at the medical device body is between the respective first/further first through-hole and the respective third/further third through-hole, wherein the respective cantilever/further cantilever in a resting position extends into the respective space/further space between the respective first/further first through-hole and the respective third/further third through-hole, wherein the respective first/further first through-hole, the respective second/further second through-hole and the respective third/further third through-hole form the respective first and second clamping mechanism. The spaces of different clamping mechanisms may form a joint space.

The additional third through-hole together with the first through-hole allow for a defined guiding of a secondary device, in particularly in case the medical device is used as a targeting device. The second through-hole is provided on the cantilever, so that deflecting the cantilever will change the inclination of the second axis along which the second through-hole in the cantilever extends. In case the cantilever is deflected, the first, second and third through-hole may be brought into alignment or may be at least brought closer to an alignment, so that a secondary device having a corresponding outer shape with a clearance fit may pass. As soon as the cantilever is released, the second through-hole leaves the aligned position and clamps the secondary device over the first and third through-hole.

According to an embodiment, the medical device further comprises a coupling interface for an implant or tool to be coupled, wherein the medical device body extends along the longitudinal extension from the first through-hole to the coupling interface for an implant or tool to be coupled.

Thus, a defined orientation and position of an implant to be implanted and the medical device can be established, particularly when using the medical device as a targeting device. The orientation of the clamping mechanism and thus the orientation of the secondary device or drill sleeve fixed therein may be established in view of and pointing to the implant coupled to the coupling interface.

According to an embodiment, an extension of a secondary device clamped in the at least one clamping mechanism is oriented toward an implant or tool coupled to the coupling interface.

Thus, a tool, e.g., a drill may be guided through the secondary device, e.g., a drill sleeve, to meet exactly a drill axis of a receptacle of the coupled implant, e.g., for preparing a path for a locking screw.

According to an embodiment, for at least one clamping mechanism an extension of the respective second axis upon deflecting of the respective cantilever with respect to an inclination approaches an extension of the respective first axis in a deflected position.

Thus, the effective cross-section through the first, second and third through-hole can be enlarged upon deflection of the cantilever for inserting a respective secondary device. The secondary device may be clamped if its outer dimension is slightly smaller than the effective cross-section of the first, second and third through-hole upon deflecting the cantilever for clearance fitting during positioning, and slightly larger in at least one direction upon un-deflecting the cantilever for fixation.

According to an embodiment, for at least one clamping mechanism the respective second axis upon deflecting into a predetermined deflected position corresponds to the respective first axis.

Thus, upon deflecting the cantilever to a particular extend a maximum effective cross-section of the first, second and third through-hole can be established for inserting the respective secondary device.

According to an embodiment, the respective first through-hole and the respective third through-hole each have a continuous constant cross-section.

Thus, a suitable and defined guiding of an inserted secondary device may be achieved. It should be noted that when providing the secondary device with a stepped cross-section, also the cross-section of the first and third through-hole may be adapted to the dimensions of the respective step of the secondary device, which allows a defined positioning of the secondary device within the through-holes in the longitudinal direction of the secondary device, by abutting a step front of the secondary device to the orifice of the respective through-hole. The second through-hole may have the dimension of the first or the third through-hole or even an intermediate dimension corresponding to a respective intermediate step of the secondary device.

According to an embodiment, the respective first through-hole and the respective third through-hole have the same cross-section.

Thus, a suitable guiding of a secondary device can be achieved combined with a flexible positioning in the longitudinal direction of the secondary device with respect to the first and third through-hole along the first axis.

According to an embodiment, the respective second through-hole has the same continuous constant cross-section as the respective first through-hole and the respective third though-hole.

Thus, a flexible fixation of the secondary device with respect to the longitudinal extension of the secondary device can be achieved along the first axis. Insertion of the secondary device into the clamping mechanism may be simplified.

According to an embodiment, sidewalls of the respective first through-hole and sidewalls of the respective third through-hole lie on the same cylindrical envelope, particularly the same elliptical cylindrical envelope, particularly the same circular cylindrical envelope.

Thus, a suitable guiding and positioning upon fixation can be achieved, while allowing a simplified manufacturing process by executing a single drill move along the first axis resulting in the first and third through-hole. Also, other cutting methods may be used, in particular when manufacturing a non-circular cross-section.

According to an embodiment, for at least one clamping mechanism sidewalls of the respective first through-hole, sidewalls of the respective third through-hole and sidewalls of the respective second through-hole upon deflecting of the respective cantilever into a predetermined deflected position lie on the same cylindrical envelope, in particular on the same elliptical cylindrical envelope, in particular on the same circular cylindrical envelope.

Thus, a suitable guiding and positioning upon fixation can be achieved, while allowing a simplified manufacturing process by executing, upon deflecting the cantilever, a single drill move along the first axis resulting in the first, second and third through-hole. The term "resting position" here is meant without a clamped shaft therein.

According to an embodiment, for at least one clamping mechanism the respective cantilever in a resting position extends orthogonal to the respective first axis.

Thus, a suitable range of deflection and relation of the effective cross-section of the first, second and third through-hole can be achieved, which is the open cross-section through all the through-holes together of one clamping mechanism.

According to an embodiment, for at least one clamping mechanism the medical device body traverse to the first axis has a respective slit extending from the respective space into the medical device body for receiving a respective fix point side of the respective cantilever.

Thus, the cantilever may be pre-manufactured and then properly fixed to the medical device body for deflecting upon operation. The slit provides a defined bearing and allows pre-positioning during manufacturing. The slit width and the thickness of the cantilever may have corresponding dimensions. The cantilever may me fixed within the slit by screws, by pressing the cantilever into the slot or by a thermal shrinking process. Gaps and intermediate spaces between the cantilever fix side and the body may be filled with a filer and sealed in order to avoid deposition of impurities.

According to an embodiment, the medical device body traverse to the respective slit has at least one threaded through bore hole extending from an outer wall of the medical device body to the respective slit for fixing the respective fix point side of the respective cantilever with a respective screw.

Thus, the cantilever can be mounted and dismounted upon fixing and releasing the screws. This allows a more flexible customizing of the medical device, as the cantilever type may be selected during manufacturing or exchanged by the user upon need.

According to an embodiment, for at least one clamping mechanism the respective space with respect to the respective first axis opposite to the respective slit has an respective opening through which a respective free side of the respective cantilever extends over an outer wall of the medical device body, wherein the respective opening is dimensioned so as to allow bringing the respective cantilever from a resting position or an un-deflected position into a deflected position upon application of a force on the respective free side and traverse to the extension direction of the respective cantilever.

Thus, the cantilever may be brought into the mounting position through the side opening opposite the fixing side during manufacturing and may be accessible on the free side for manual deflection upon operation.

According to an embodiment, the respective space through which a respective free side of the respective cantilever extends over an outer wall of the medical device body, has a stopper face being adapted for limiting a deflection and defining the deflected position, wherein in the respective defined deflected position sidewalls of the respective first through-hole, sidewalls of the respective third through-hole and sidewalls of the respective second through-hole lie on the same or lie closer to a cylindrical envelope.

Thus, the deflected position of the cantilever resulting in e.g., the largest effective cross-section of all through-holes, in particular the first and second through-hole, may be predetermined by the stopper face, where the cantilever abuts upon deflection.

According to an embodiment, for at least one clamping mechanism the respective cantilever at a respective free side of the respective cantilever, in particular at a respective free side portion of the respective cantilever extending over the outer wall of the medical device body has a respective finger grip arrangement.

Thus, the operation of the clamping mechanism may be made safer and more comfortable. The finger grip arrangement may be a detention or a portion with a fluting, a grid or any other structure which avoids or reduces the risk of a finger for slipping out of or away from the finger grip.

According to an embodiment, the first axis and the further first axis are inclined with respect to each other.

Thus, different orientations of secondary devices fixed in the respective clamping mechanisms may be achieved. This may be useful when implanting an implant which requires two or more locking screws. Such medical device may allow positioning of e.g., two or more drill sleeves at the same time, so that multiple drill processes can be carried out without the need for repositioning the medical device, in particular when used as a targeting device. The term "inclined" means that the axes if laying in a plane include an angle, and if not laying in a plane are not parallel to each other.

According to an embodiment, there is provided a surgical targeting system having a medical device as described above as a targeting device and a secondary device to be targeted, which secondary device has a shaft which for at least one clamping mechanism is adapted to clearance fit the respective first through-hole, the respective second through-hole and the respective third through-hole when the respective cantilever is in a predetermined deflected position, and to be blocked in the respective first through-hole, the respective second through-hole and the respective third through-hole when the respective cantilever is in a less deflected or resting clamping position between the predetermined deflected position and the resting position so that the secondary device is fixed in a targeted position of the secondary device over the medical device.

Thus, a targeting system may be provided which allows for an easy use and reliable targeting for setting e.g., one or more locking screws for an implant.

According to an embodiment, there is provided a method for manufacturing a clamping mechanism in a medical device for fixing a secondary device to be connected to said medical device, wherein the method comprises providing a medical device body with a space between a position intended for a first through-hole and a position intended for a third through-hole, and a cantilever extending into a resting position into the space traverse to a first axis extending from the position intended for a first through-hole and a position intended for a third through-hole; elastically deflecting the cantilever from a resting position into a predetermined deflected position by applying a force on a free end of the cantilever along the first axis; cutting by a single cylinder cutting process along the first axis subsequently a first through-hole through the medical device body, a second through-hole through the cantilever and a third through-hole through the medical device body, so that in the deflected position sidewalls of the first through-hole, sidewalls of the third through-hole and sidewalls of the second through-hole lie on the same circular cylindrical envelope.

Thus, a simplified manufacturing process may be provided for manufacturing a medical device as describe above. The through-hole in the cantilever is manufactured while the cantilever is deflected, so that any axial mismatch of the through-holes may be prevented. The term "single cylinder cutting process" is to be understood as a cutting process which forms a (not mandatorily circular) cylinder envelope along an axis, here the first axis. The single cylinder cutting process may be realized by a single drilling process, which for technical reason may include multiple forth and back movements of the drilling tool, but result in a circular cylinder envelope, here corresponding to the first, second and second through-hole sidewalls. It should be noted that the method also applies for clamping mechanisms which have only a first and second through-hole. In such a method the cantilever is deflected over the first through-hole and a single cutting process is applied.

According to an embodiment, the method further comprises releasing the cantilever so that the cantilever elastically moves back to the resting position.

According to an embodiment, there is provided a method for fixing a secondary device, such as a drill sleeve, to the medical device including the steps of moving the cantilever from a resting position to a deflected position so that the second through-hole on the cantilever aligns with the first and third through-holes on the medical device; inserting the secondary device through the first, second and third through-holes; and returning the cantilever to the resting position to retain the secondary device within the first, second and third through-holes.

According to another embodiment, there is provided a method for fixing first and second secondary devices, such as two drill sleeves, to a medical device including the steps of moving a cantilever (first cantilever) from a first resting position to a first deflected position; inserting a first secondary device through the medical device and the cantilever (first cantilever) in the first deflected position; returning the cantilever (first cantilever) to the first resting position to connect the first secondary device to the medical device; moving a further cantilever (second cantilever) from a second resting position to a second deflected position; inserting a second secondary device through the medical device and the further cantilever (second cantilever) in the second deflected position; and returning the further cantilever (second cantilever) to the second resting position to connect the second secondary device to the medical device.

It should be noted that the above-described embodiments may also be combined and in a combined form provide a synergetic technical effect and synergetic benefits which go beyond the sum of the single technical effects and benefits.

### Brief Description of the Figures

The invention will be described by way of the following drawings:
- Figure 1:: illustrates a perspective view of a medical device with a clamping mechanism according to the prior art.
- Figure 2:: illustrates a perspective view of an anatomy having implanted an implant with a medical device.
- Figure 3:: illustrates a top view of a medical device with a clamping mechanism according to the prior art.
- Figure 4:: illustrates a perspective view of a medical device with a clamping mechanism according to an exemplary embodiment.
- Figure 5:: shows a side view of the medical device of Figure 4 with a clamping mechanism according to an exemplary embodiment.
- Figure 6:: shows a cross-sectional front view of the medical device of Figure 4 with a clamping mechanism according to an exemplary embodiment with a first and second through-hole in the medical device body and a second through-hole in the cantilever.
- Figure 7:: shows a cross-sectional front view of the medical device with a clamping mechanism according to a further exemplary embodiment with a first through-hole in the medical device body and a second through-hole in the cantilever.
- Figure 8:: illustrates a perspective view of a medical device with a first and second clamping mechanism according to an exemplary embodiment.
- Figure 9:: shows a cross-sectional front view of a medical device according to an exemplary embodiment with a plurality of clamping mechanisms each with a first and second through-hole in the medical device body and a second through-hole in the cantilever.
- Figure 10:: shows a cross-sectional front view of a medical device according to an exemplary embodiment with a plurality of clamping mechanisms each with a first through-hole in the medical device body and a second through-hole in the cantilever.
- Figure 11a and 11b:: show a schematic principle of a locking (Figure 11a) and releasing (Figure 11b) of a shaft of a secondary device with respect to the medical device according to an exemplary embodiment.
- Figure 12a and 12b:: show a schematic principle of a resting position (Figure 12a) and a deflected position (Figure 12b) of the cantilever for locking and releasing of a shaft (here not shown) of a secondary device with respect to the medical device according to an exemplary embodiment.
- Figure 13a and 13b:: show a schematic principle of a locking (Figure 13a) and releasing (Figure 13b) of a shaft of a secondary device with respect to the medical device (here only the cantilever thereof is illustrated) according to a further exemplary embodiment.
- Figure 14:: illustrates the understanding of the terminology of a cross-sectional projection in general and a maximum cross-sectional projection in particular.
- Figure 15:: illustrates a schematic flow chart of the method for manufacturing of the medical device with a clamping mechanism according to an exemplary embodiment.

It should be noted that same or similar reference numerals illustrate same or similar components. Along these Figures exemplary embodiments of the invention will be described as follows.

### Detailed Description of Exemplary Embodiments

The invention will be described along exemplary embodiments, which are illustrated in the above references figures and will be described in detail in the following.

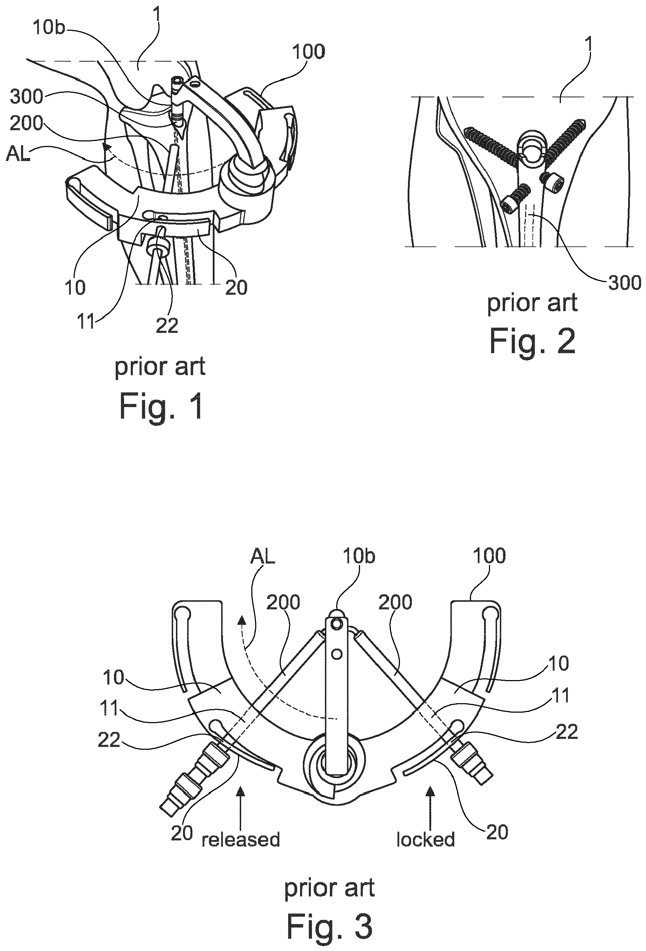

Figure 1 illustrates a perspective view of a medical device with a clamping mechanism according to the prior art. For implanting a rod implant 300 or nail into a medullary channel of a bone 1, the rod implant or nail 300 can be coupled to a medical targeting device 100. The medical targeting device 100 has a body 10 and a coupling interface 10b for coupling the implant 300. The medical device 100 may have a number of through-holes 11 which may serve for targeting a tool 200, e.g., a targeting drill sleeve, for applying a locking screw to the implant to fix the position of the implant 300 within the patient's anatomy 1. The medical device 100 may have a number of cantilevers 20 each extending over the respective through-hole 11 into a longitudinal direction of the medical device. The cantilever usually extends along a longitudinal extension AL of the medical device 100 and may be bent toward the body 10 of the medical device 100. The cantilever may also have a through-hole 22, which lies over the respective through-hole 11 of the body 10 of the medical device 100. The through-hole 11 in the body 10, in the following also referred to as the first through-hole 10, of the medical device 100 may have an axis, in the following referred to as first axis, along which the first through-hole 11 in the body 11 extends. The through-hole 22 in the cantilever 20, in the following also referred to as the second through-hole 22, may have an axis, in the following referred to as second axis, along which the second through-hole 22 in the body 10 extends, which is inclined over the first axis along which the first through-hole 11 extends if the cantilever 20 is not bent, but is not inclined over the first axis along which the first through-hole 11 extends if the cantilever 20 is bent toward the body 10 of the medical device 100. In the bent state, a secondary device like a drill sleeve 200 having a corresponding diameter of the first through-hole 11 and the second through-hole 22 may be put through the first through-hole 11 and the second through-hole 22 due to the not inclined or less inclined extending axes of the first through-hole 11 and the second through-hole 22 upon deflected cantilever 20. If the cantilever 20 returns to a state where the inclination between the extending axes of the first through-hole 11 and the second through-hole 22 increases, the drill sleeve 200 will be clamped and thus fixed due to the canting and clamping. Thus, the e.g., drill sleeve 200 is fixed over the medical device 100 and consequently over the implant 300 in a defined orientation, where a drill trajectory of the drill sleeve 200 points toward a respective threaded hole (not shown) of the implant 300. This allows to drill a hole into the patient's anatomy, e.g., a bone, so that locking screws may be implemented to fix the implant 300 to the bone 1.

Figure 2 illustrates a perspective view of an anatomy 1 having implanted an implant 300. The implant 300, e.g., an implant rod or a nail may be fixed within a bone of the human's anatomy 1 by inserting locking screws through the bone 1 and the implant rod 300 or nail in order to fix the implant rod or nail to the bone.

Figure 3 illustrates a top view of a medical device according to the prior art with a clamping mechanism according to the prior art. The elements are the same as described for figure 1. Figure 3 illustrates the left cantilever 20 in a bent position where the secondary device, e.g., a drill sleeve 200 is in a released state where it can be moved along the longitudinal extension of device 200 through the first through-hole 11 in the body 10 and the second through-hole 22 in the cantilever 20. As soon as the cantilever 20 returns toward its unbent position, as illustrated on the right side, the secondary device, e.g., a drill sleeve 200 is in a fixed or locked state where it cannot be moved along the longitudinal extension of device 200 any longer through the first through-hole 11 in the body 10 and the second through-hole 22 in the cantilever 20. The drill sleeve 200 in this state is fixed and oriented toward the (here not illustrated) implant, which can be coupled to the coupling portion 10b.

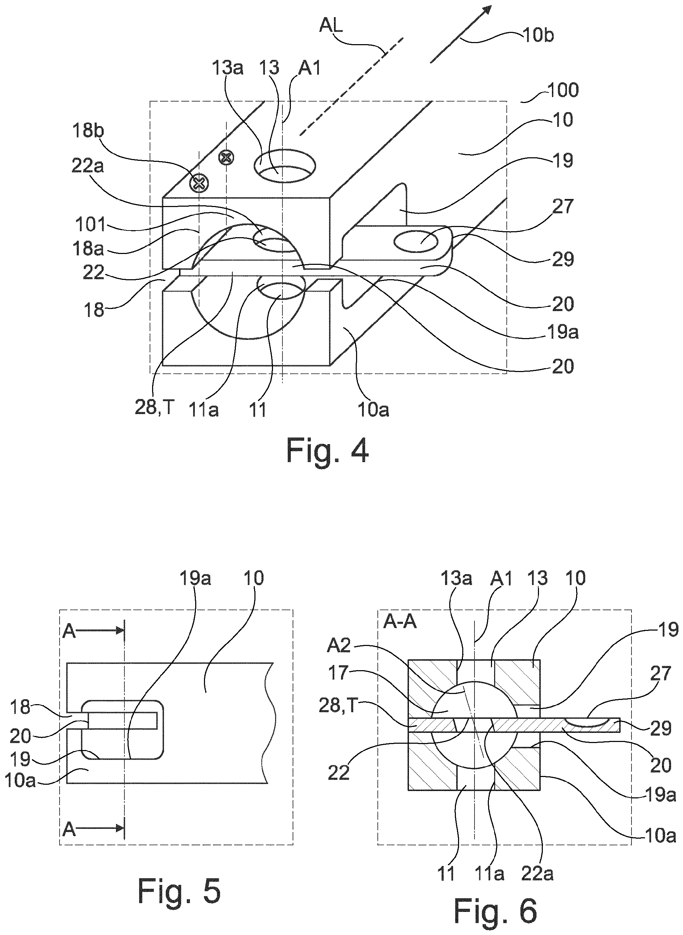

Figure 4 illustrates a perspective view of a medical device 100 with a clamping mechanism 101 according to an embodiment of the invention. Figure 5 shows a side view of the medical device of Figure 4 with a clamping mechanism according to an exemplary embodiment. Figure 6 shows a cross-sectional front view of the medical device of Figure 4 and 5 with a clamping mechanism according to an exemplary embodiment. Figure 4, Figure 5 and Figure 6 illustrate the medical device 100 having a clamping mechanism 101 for fixing a secondary device 200 (not illustrated here) with respect to the medical device 100. The medical device 100 has a medical device body 10 and a cantilever 20, which is fixed to the body 10 in a deflectable manner. The cantilever may be manufactured in a separate piece and then fixed to the body 10, e.g., by screws 18b along a fixing axis 18a, or may be formed with the body as a single piece, e.g., manufactured by a milling process. The cantilever may be formed in total of a material which allows a deflecting or may have a material section between the fixation and the second through-hole 22 allowing the deflecting, whereas the section with the second through-hole is formed by a material which is more rigid than the deflectable section.

The medical device body 10 comprises a first through-hole 11 extending along a first axis A1, and a third through-hole 13 which is aligned to the first through-hole 11 along the first axis A1, and a space 17 at the medical device body 10 between the first through-hole 11 and the third through-hole 13. This space allows the deflecting displacement of the cantilever 20. The space at the medical device body may be a space formed within the medical device body or a space being partially surrounded by the medical device body. In case it is referred to a slit in the space, it is also meant a slit in the medical device body at a face of the medical device body, which forms at least one boundary of the space.

As the clamping mechanisms as illustrated in e.g., Figures 4, 5, 6 and 8, compared to the prior art clamping mechanism illustrated in claims 1, 2 and 3, have an additional through-hole 13, the guidance of e.g., a drilling sleeve (not illustrated here) can be improved. Further, the orientation can be defined much more exactly, as a drilling sleeve is supported by side wall 11a of the first through-hole 11 and side wall 13a of the third through-hole 13, upon fixation of the drilling sleeve by a side wall 22a of the second through-hole 22 of the cantilever 20.

The cantilever 20 in a resting position extends into the space between the first through-hole 11 and the third through-hole 13 traverse to the first axis A1. The resting position of a cantilever is to be understood as the position where no forces act on the cantilever. The resting position, if not declared otherwise, is meant without a clamped shaft therein. It is understood that the gravity force acting on the cantilever does not move the cantilever out of the resting position. In comparison thereto, a deflected or bent position is a position of the cantilever, which is achieved by a force acting on the cantilever traverse to the extension direction of the cantilever and distant from the cantilever fixing point, i.e., a torque, and which moves the cantilever out of the resting position. As a cantilever intrinsically is mounted on only one side, a torque will deflect the cantilever and thus will also incline upon deflection the second axis with respect to the extension of the second axis in the resting position. As traverse it is to be understood, that the extension of the cantilever is not along the aligning axis of the both through-holes, but intersects the aligning axis. Traverse may also be orthogonal.

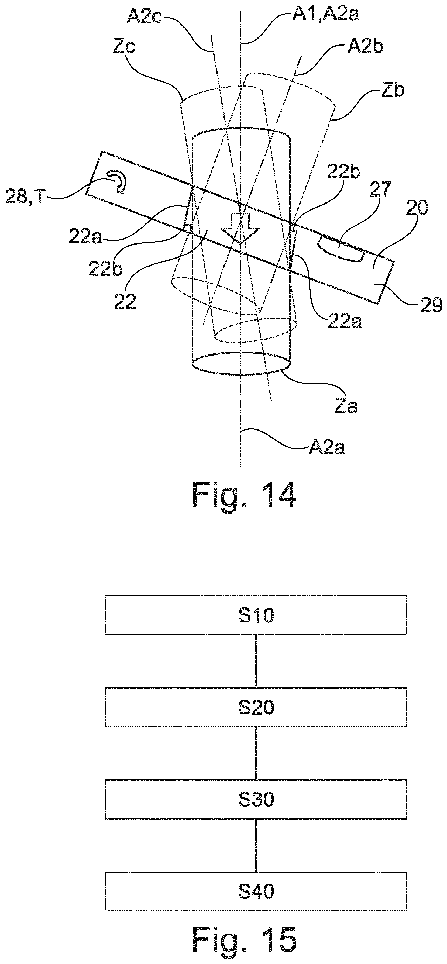

The cantilever 20 has a second through-hole 22, wherein the second through-hole 22 in the resting position has a maximum cross-sectional projection along a second axis A2 being inclined to the first axis A1. A cross-sectional projection of a through-hole along an axis means a projection of the cross-section, which is open at any depth of the through-hole along said axis. A maximum cross-sectional projection can be considered as a cross-section of a (not mandatorily circular) cylinder along said axis with a maximal possible cross-section, which still passes the through-hole. Figure 14 illustrates the maximum cross-section for a through-hole 22 which does not have a cylindrical cross-section, but has a clamping section 22b, which protrudes inwardly into the through-hole 22. The projection with the maximum cross-section corresponds to a view along the axis A1, A2a of a maximum cross-section cylinder Za. As can be seen from Figure 14, other viewing directions along an axis A2b and A2c correspond to cylinders Zb and Zc with only a lower dimension and cross-section.

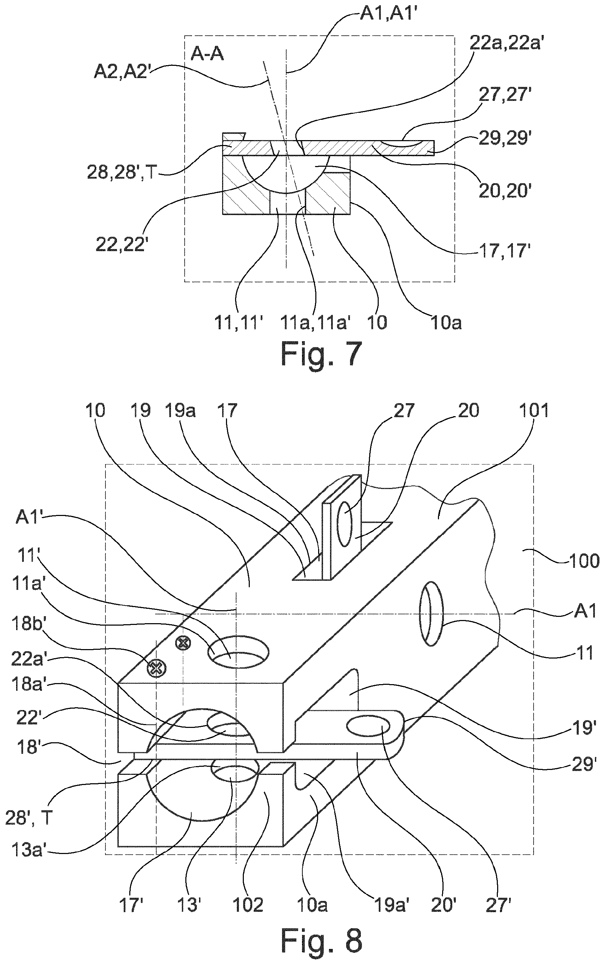

The first and second through-hole 11, 22 may e.g., be manufactured by deflecting the cantilever 20 toward the location of the first through-hole 11 and then drilling the first through-hole 11 and the second through-hole 22 together, so that they have the same aligning axis A1 and A2. For the third through-hole, the same applies for the first through-hole. If releasing the cantilever 20, the cantilever returns to its resting position, so that the axis A2 along which the second through-hole 22 aligns inclines to the first axis A1 along which the first through-hole 11 aligns, as illustrated in figure 7. Inclined is to be understood in that the axes if laying in a plane include an angle, and if not laying in a plane are not parallel to each other. The first through-hole 11, the second through-hole 22 and the third through-hole 13 form the clamping mechanism 101.

The cantilever 20 can be manufactured as a separate piece as described above and can be inserted into a slit 18 formed in the body 10 of the medical device 100. The width of the slit 18 and the thickness of the cantilever 20 may have the same dimension. The cantilever 20 may be fixed on its fixing side 28 to the slit 18 by screws 18b. As an alternative, the cantilever can be pressed into the slit or inserted by thermic shrinking, where the cantilever is cooled down and inserted into a warmer body 10 with slit 18, so that the cantilever upon thermal expanding press fits into the slit 18.

The medical device body 10 may have formed in its outer wall 10a an opening 19, which allows the cantilever 20 to bend or to deflect around its torque point or axis close to the fixing point of the cantilever 20. The opening 19 may have a deflective stopper 19a, which stops deflection of the cantilever at a position where the first through-hole 11, the second through-hole 22 and the third through-hole 13 may align with respect to their extension through the respective element. In this position, regardless of any possible inwardly protruding clamping portions 22b, the wall 11a of the first through-hole 11, the wall 22a of the second through-hole 22 and the wall 13a of the third through-hole 13 may be on the same cylinder barrel surface, in particular on the same elliptical cylinder barrel surface, in particular on the same circular cylinder barrel surface of a maximum cross-section cylinder Za.

When inserting a secondary device 200 like a drill sleeve into the first through-hole 11, the second through-hole 22 and the third through-hole 13, a shaft 210 (not shown in Figures 4, 5 and 6) laterally abuts the wall 11a of the first through-hole 11, the wall 22a of the second through-hole 22 and the wall 13a of the third through-hole 13. In case no third through-hole is provided, as described later, the shaft 210 of the secondary device only laterally abuts the wall 11a of the first through-hole 11 and the wall 22a of the second through-hole 22.

The free side 29 of the cantilever 20 may be provided with a finger grip arrangement 29. Finger grip arrangement may be a detention or a portion with a fluting, a grid or any other structure which avoids or reduces the risk of a finger for slipping out of the finger grip.

Figure 7 shows a cross-sectional front view of the medical device with a clamping mechanism according to a further exemplary embodiment with a first through-hole 11 in the medical device body 10 and a second through-hole 22 in the cantilever 20. The medical device 100 has a clamping mechanism 101 for fixing a secondary device 200 (not illustrated in Figure 7) to be connected to the medical device 100. As described with respect to Figures 4, 5 and 6, the medical device comprises a medical device body 10 having a longitudinal extension AL, and a cantilever 20. A longitudinal extension with this respect means an extension of the medical device body from a location where the first through-hole 11 is located and a further inventory of the medical device, which may be a further clamping or a coupling interface 10b for an implant or tool 300 to which the secondary device 200 is oriented upon clamping. This may correspond to the extension along the largest dimension of the medical device 100. This may also include an extension along a bended path for a curved body 10 of a medical device 100 or even a branched path. The longitudinal extension can be understood as a path the structural material of the body follows. The medical device body 10 comprises the first through-hole 11 extending along the first axis A1, and the space 17 at the medical device body 10 in front of the first through-hole 11. The space 17 at the medical device body 10 may be a space formed within the medical device body 10, as illustrated in Figures 4, 5 and 6, or a space being partially surrounded by the medical device body 10, as illustrated in Figure 7.

The cantilever 20 in a resting position extends into the space 17 in front of the first through-hole 11 traverse to the first axis A1 and traverse to the longitudinal extension AL of the medical device body and has a second through-hole 22. The second through-hole 22 in the resting position has a maximum cross-sectional projection along a second axis A2 being inclined to the first axis A1. The first through-hole 11 and the second through-hole 22 form the at least one clamping mechanism 101. References without an apostrophe refer to an item of the medical device 100 or clamping mechanism 101, 102 in general as well as the first clamping mechanism 101, whereas references with apostrophe refer to the further or second clamping mechanism 102, as illustrated in, e.g., Figure 8.

Figure 8 illustrates a perspective view of a medical device with a first and second clamping mechanism according to an exemplary embodiment. For the second clamping mechanism 101 in the rear section (with the references without apostrophe), the description of Figures 4, 5 and 6 applies. The medical device 100 has a further clamping mechanism 102. For this purpose, the medical device 100 comprises a further cantilever 20'. The medical device body 10 has a further first through-hole 11' extending along a further first axis A1', a further third through-hole 13' which is aligned to the further first through-hole 11' along the further first axis A1', and a further space 17' at the medical device body 10 between the further first through-hole 11' and the further third through-hole 13'. The further cantilever 20' in a resting position extends into the further space 17' between the further first through-hole 11' and the further third through-hole 13' traverse to the further first axis A1' and has a further second through-hole 22'. The further second through-hole 22' in the resting position has a maximum cross-sectional projection along a further second axis A2' being inclined to the further first axis A1'. The further first through-hole 11', the further second through-hole 22' and the further third through-hole 13' form the second clamping mechanism 102.

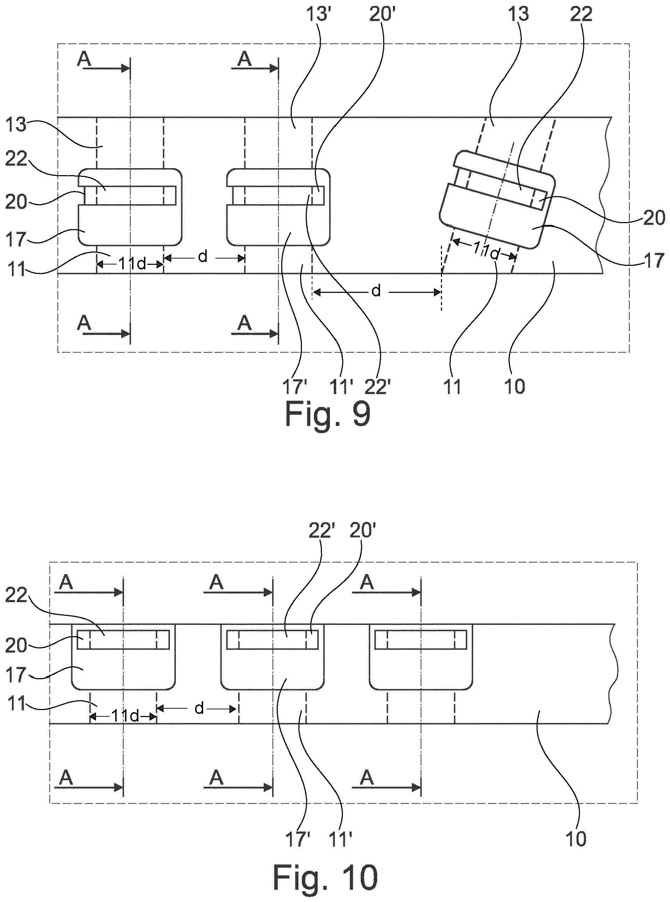

Although both clamping mechanisms 101 and 102 have an orientation orthogonal to each other, also any other orientations are possible, as will be described with respect to Figure 9 and 10. It is also possible to provide more than two clamping mechanisms, as will also be illustrated in Figures 9 and 10.

Figure 9 shows a cross-sectional front view of a medical device according to an exemplary embodiment with a plurality of clamping mechanisms each with a first through-hole 11 and third through-hole 13 in the medical device body 10 and a second through-hole 22 in the cantilever 20. The description of Figures 4, 5 and 6 applies accordingly also for Figure 9. As can be seen from Figure 9, the clamping mechanisms can have a different orientation according to the requirements at the medical device 100. It should be understood that the extension of the body 10 may also be along a curved or bent line, e.g., in order to orient the aligning axes A1 toward an implant (not shown here) coupled to the medical device 100. It should be noted that the spaces 17 of two or more clamping mechanisms may be provided by a joint space.

Figure 10 shows a cross-sectional front view of a medical device according to an exemplary embodiment with a plurality of clamping mechanisms each with a first through-hole 11 in the medical device body 10 and a second through-hole 22 in the cantilever 20. The description of Figure 7 here also applies for all three illustrated clamping mechanisms in Figure 10. It should be understood that the extension of the body 10 may also be along a curved or bent or branched line, e.g., in order to orient the aligning axes A1 toward an implant (not shown here) coupled to the medical device 100.

As the clamping mechanisms as illustrated in e.g., Figures 4, 7, 8, and 10 compared to the prior art clamping mechanism illustrated in claims 1, 2 and 3, have a cantilever 20 extending traverse to the longitudinal extension AL of the medical device body 10, the cantilever no longer occupies space between adjacent clamping mechanisms as in Figures 1, 2 and 3. This allows positioning of adjacent clamping mechanisms closer to each other, without losing the functionality. In particular, when there is a need for preparing two drilling holes close to each other, drilling sleeves may be provided therefore at the same time, so that no repositioning of the medical device 100 is required.

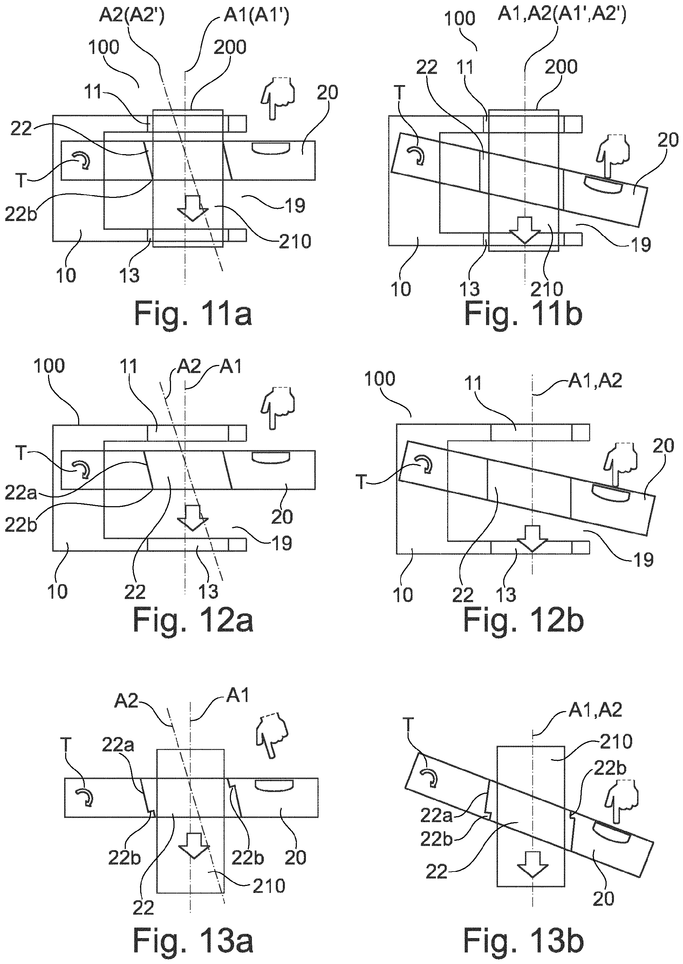

Figure 11a and 11b show a schematic principle of a locking (Figure 11a) and releasing (Figure 11b) of a shaft 210 of a secondary device 200 with respect to the medical device 100 according to an exemplary embodiment. In Figure 11a, the shaft 210 is clamped between the wall sections 11a, 22a, 13a of the first through-hole 11, the second through-hole 22 and the third through-hole 13, respectively. It should be noted that the gaps between the shaft 210 and the first and third through-hole walls are only for illustrative purposes and should illustrate that the shaft 210 and the respective through-hole 11, 13 are separate elements. The shaft of each of the through-holes has a clearance fit. In the clamped position illustrated in Figure 11a one side wall portion of the first and third through-hole also abuts the shaft on one side, whereas a wall section of the second through-hole 22 abuts the shaft 210 on the opposing side. This means that upon clamping the shaft does not have a clearance fit over all through-holes together, but only over each separate through-hole alone. Upon pressing the cantilever 20 (Figure 11b) the second through-hole 22 of the cantilever 20 releases shaft 210, as it no longer abuts the shaft 210, so that the shaft can move with respect to the body 10 and then has a clearance fit over all through-holes together. Although the cantilever is illustrated as rotating around its fixed side 28 and torque point T, it should be understood that the deflection may also be realized by a bending of the cantilever with a clamped fixed side 28.

Figure 12a and 12b show a schematic principle of a resting position (Figure 12a) and a deflected position (Figure 12b) of the cantilever for locking and releasing of a shaft (here not shown) of a secondary device 200 with respect to the medical device 100 according to an exemplary embodiment. For Figure 12a and 12b, the same applies as described with respect to Figures 11a and 11b.

Figure 13a and 13b show a schematic principle of locking (Figure 13a) and releasing (Figure 13b) of a shaft of a secondary device with respect to the medical device (here only the cantilever thereof is illustrated) according to a further exemplary embodiment, where the inner wall 22a of the second through-hole 22 is provided with protrusions 22b, which form a clamping portion. This portion of the wall, be it in the form of an explicit protrusion as illustrated in Figure 12a and 12b, or be it in a straight wall as illustrated e.g., in Figure 11a and 11b as well as 12a and 12b, may be hardened. The protrusions may have a form of a blade which may cut into the shaft 210. In case the secondary device 200 is provided as a single use device, a cut in the shaft 210 executed by a blade of the protrusion 22b may be acceptable or desired, in order to increase the fixation.

Figure 14 illustrates the understanding of the terminology of a cross-sectional projection in general and a maximum cross-sectional projection in particular. Figure 14 illustrates the maximum cross-section for a through-hole 22 which does not have a cylindrical cross-section, but has a clamping section 22b, which protrudes into the through-hole 22. The same description however applies to a second through-hole 22 with a straight wall section 22a without an explicit protrusion on clamping portion 22b. The projection with the maximum cross-section corresponds to a view along the axis A1, A2a of a maximum cross-section cylinder Za. As can be seen from Figure 14, other viewing directions along an axis A2b and A2c correspond to cylinders Zb and Zc with only a lower diameter and cross-section. With the corresponding orientation of axis A1 and A2a the maximum opening projection is achieved which is larger than an opening projection with aligned axes A1 and A2b or A1 and A2c. The maximum opening cross-section projection allows insertion of the shaft 210 as outlined above so that it can be moved along the axis A1.

Figure 15 illustrates a schematic flow chart of the method for manufacturing of the medical device with a clamping mechanism according to an exemplary embodiment. After providing a medical device body 10 in S10, before having manufactured through-holes 11, 22, 13, the cantilever 20 is elastically deflected toward the location of the first through-hole 11 in S20. Then the first through-hole 11 and the second through-hole 22, and if a third through-hole is provided, also the third through-hole are drilled or cut in one step, which means that their bore follows the same cylinder barrel shape, so that they have the same aligning axis A1 and A2. Then the cantilever 20 is released from the deflected or bent position into the relaxed position where the first axis A1 of the first through-hole 11, and if a third through-hole 13 is provided also of the third through-hole 13 is inclined over the second axis A2 of the second through-hole 22.

A method of fixing a secondary device 200 to a medical device 100 according to the present invention will now be described. The method generally includes moving the cantilever 20 from a resting position to a deflected position so that the second through-hole 22 on the cantilever 20 aligns with the first and third through-holes 11 and 13 on the medical device 100; inserting the secondary device 200 through the first, second and third through-holes 11, 22, and 13, respectively; and returning the cantilever 20 to the resting position to retain the secondary device 200 within the first, second and third through-holes 11, 22, and 13, respectively.

The secondary device 200 may be a drill sleeve. In that case, a targeting arm may be used to provide a targeting geometry for the drill sleeve. The targeting arm may be connected to the end of an implant, which may be an intramedullary rod implant to be inserted into an intramedullary canal of a long bone, such as the tibia or femur. The drill sleeve, using the general method described above, will be clamped in place for X-ray imaging or other preparations before continuing implantation.

Again, this method is beneficial for defining the orientation of the secondary device 200 because the drilling sleeve is supported by side wall 11a of the first through-hole 11 and side wall 13a of the third through-hole 13, upon fixation of the drilling sleeve by a side wall 22a of the second through-hole 22 of the cantilever 20.

It is understood that this method of fixing a secondary device 200 to a medical device 100 is not limited to first, second, and third through-holes 11, 22, and 13, respectively. There may be less than or more than three holes in which the secondary device 200 is inserted. Additionally, there may be more than one cantilever 20 and more than one secondary device 200 for insertion.

A method of fixing first and second secondary devices 200 to a medical device 100 according to the present invention will now be described. The method generally includes a first clamping mechanism 101 including moving a cantilever (first cantilever) 20 from a first resting position to a first deflected position; inserting a first secondary device 200 through the medical device 100 and the cantilever (first cantilever) 20 in the first deflected position; and returning the cantilever (first cantilever) 20 to the first resting position to connect the first secondary device 200 to the medical device 100. Additionally, the method generally includes a second clamping mechanism 102 including moving a further cantilever (second cantilever) 20' from a second resting position to a second deflected position; inserting a second secondary device 200 through the medical device 100 and the further cantilever (second cantilever) 20' in the second deflected position; and returning the further cantilever (second cantilever) 20' to the second resting position to connect the second secondary device 200 to the medical device 100.

This method may include inserting the first secondary device 200 through the first, second and third through-holes 11, 22, and 13, respectively and the second secondary device through the further first, further second and further third through-holes 11', 22', and 13', respectively.

The first and secondary devices 200 may be two drill sleeves. Again, this method is beneficial when using one or more drill sleeves at the same time because multiple drill processes can be carried out without the need for repositioning the medical device 100. This method may be used in conjunction with a targeting device.

It is understood that this method of fixing first and second secondary devices 200 to a medical device 100 is not limited to two secondary devices. There may be more than two cantilevers 20 to permit the fixation of more than two secondary devices. For example, the method may additionally include moving a third cantilever from a third resting position to a third deflected position; inserting a third secondary device 200 through the medical device 100 and the third cantilever in the third deflected position; and returning the third cantilever to the third resting position to connect the third secondary device 200 to the medical device 100.

### Reference numbers

- 1: patient anatomy
- 10: medical device body
- 10a: outer wall of medical device body
- 10b: coupling interface for coupling an implant/tool
- 11: first through-hole in medical device body
- 11a: side wall of first through-hole
- 11d: diameter of the first through-hole
- 13: third through-hole in medical device body
- 13a: side wall of third through-hole
- 17: space between first and third through-hole in medical device body
- 18: slit in medical device body for mounting fix side of cantilever
- 18a: (threaded) through bore for receiving pin/screw for fixing fix point side of cantilever
- 18b: pin, bolt, rivet, screw for fixing fix point side of cantilever to medical device body
- 19: opening opposite of slit
- 19a: deflective stopper/abutment for cantilever
- 20: cantilever
- 22: second through-hole in cantilever
- 22a: side wall of second through-hole
- 22b: clamping portion
- 27: finger grip arrangement, detention, fluting, grid for fractioned finger resting
- 28: fix point side of cantilever
- 29: free side of cantilever
- 100: medical device
- 101: first clamping mechanism
- 102: second clamping mechanism
- 200: secondary device/drill sleeve
- 210: shaft of secondary device
- 300: implant/tool coupled/to be coupled to medical device
- A, A1: first axis along which first and third through-hole align
- A2: second axis along which second through-hole aligns
- A2a: cylinder axis of maximum cross-section cylinder Za
- A2b: cylinder axis of lower cross-section cylinder Zb
- A2c: cylinder axis of lower cross-section cylinder Zv
- AL: longitudinal extension of medical device/medical device body
- d: lateral distance between adjacent first through-holes in medical body
- S10: providing medical device body (before having manufactures through-holes)
- S20: elastically deflecting cantilever
- S30: cutting, drilling of the through-holes
- S40: releasing cantilever from deflected/bended position
- T: torque point
- Za: maximum cross-section cylinder
- Zb: lower cross-section cylinder
- Zc: lower cross-section cylinder

References without an apostrophe refer to an item of the medical device or clamping mechanism in general as well as the first clamping mechanism, whereas references with apostrophe refer to the further or second clamping mechanism.

## Claims

1. Medical device with at least one clamping mechanism (101, 102) for fixing a secondary device (200) to be connected to said medical device (100), the medical device comprises
a medical device body (10), and
a cantilever (20),
wherein the medical device body (10) comprises:
a first through-hole (11) extending along a first axis (A1),
a third through-hole (13) which is aligned to the first through-hole (11) along the first axis (A1), and
a space (17) at the medical device body (10) between the first through-hole (11) and the third through-hole (13),
wherein the cantilever (20) in a resting position extends into the space (17) between the first through-hole (11) and the third through-hole (13) traverse to the first axis (A1) and has a second through-hole (22), wherein the second through-hole (22) in the resting position has a maximum cross-sectional projection along a second axis (A2) being inclined to the first axis (A1)
wherein the first through-hole (11), the second through-hole (22) and the third through-hole (13) form the at least one clamping mechanism (101, 102).

2. Medical device according to claim 1, wherein the medical device comprises
a further cantilever (20'),
wherein the medical device body (10) comprises:
a further first through-hole (11') extending along a further first axis (A1'),
a further third through-hole (13') which is aligned to the further first through-hole (11') along the further first axis (A1'), and
a further space (17') at the medical device body (10) between the further first through-hole (11') and the further third through-hole (13'),
wherein the further cantilever (20') in a resting position extends into the further space (17') between the further first through-hole (11') and the further third through-hole (13') traverse to the further first axis (A1') and has a further second through-hole (22'), wherein the further second through-hole (22') in the resting position has a maximum cross-sectional projection along a further second axis (A2') being inclined to the further first axis (A1')
wherein the further first through-hole (11'), the further second through-hole (22') and the further third through-hole (13') form a second clamping mechanism (102).

3. Medical device with at least one clamping mechanism (101, 102) for fixing a secondary device (200) to be connected to said medical device (100), the medical device comprises
a medical device body (10) having a longitudinal extension (AL), and
a cantilever (20),
wherein the medical device body (10) comprises:
a first through-hole (11) extending along a first axis (A1), and
a space (17) at the medical device body (10) in front of the first through-hole (11),
wherein the cantilever (20) in a resting position extends into the space (17) in front of the first through-hole (11) traverse to the first axis (A1) and traverse to the longitudinal extension (AL) of the medical device body and has a second through-hole (22), wherein the second through-hole (22) in the resting position has a maximum cross-sectional projection along a second axis (A2) being inclined to the first axis (A1),
wherein the first through-hole (11) and the second through-hole (22) form the at least one clamping mechanism (101, 102).

4. Medical device according to claim 3, wherein the medical device comprises
a further cantilever (20'),
wherein the medical device body (10) comprises:
a further first through-hole (11') extending along a further first axis (A1'), and
a further space (17') at the medical device body (10) in front of the further first through-hole (11'),
wherein the further cantilever (20') in a resting position extends into the further space (20') in front of the further first through-hole (11') traverse to the further first axis (A1') and traverse to the longitudinal extension (AL) of the medical device body (10) and has a further second through-hole (22'), wherein the further second through-hole (22') in the resting position has a maximum cross-sectional projection along a further second axis (A2') being inclined to the further first axis (A1'),
wherein the further first through-hole (11') and the further second through-hole (22') form a second clamping mechanism (102).

5. Medical device according to any one of claims 2 and 4, wherein a minimum lateral distance (d) between the first through-hole (11) and the further first through-hole (11') is less than three times of the minimum diameter (11d) of the first through-hole (11), in particular less than two times of the minimum diameter (11d) of the first through-hole (11), in particular less than the minimum diameter (11d) of the first through-hole (11), in particular less than half of the minimum diameter (11d) of the first through-hole (11).

6. Medical device according to any of claims 3 to 5, wherein the medical device body (10) comprises at least one of a third and further third through-hole (13, 13') which is aligned to the respective first/further first through-hole (11, 11') along the respective first/further first axis (A1, A1'), wherein the respective space/further space (17, 17') at the medical device body (10) is between the respective first/further first through-hole (11) and the respective third/further third through-hole (13, 13'), wherein the respective cantilever/further cantilever (20, 20') in a resting position extends into the respective space/further space (20, 20') between the respective first/further first through-hole (11, 11') and the respective third/further third through-hole (13, 13'), wherein the respective first/further first through-hole (11, 11'), the respective second/further second through-hole (22, 22') and the respective third/further third through-hole (13, 13') form the respective first and second clamping mechanism (101, 102).

7. Medical device according to any one of claims 1 to 6, wherein for at least one clamping mechanism (101, 102) an extension of the respective second axis (A2, A2') upon deflecting of the respective cantilever (20, 20') with respect to an inclination approaches an extension of the respective first axis (A1, A1') in a deflected position.

8. Medical device according to any of claims 1 to 7, wherein for at least one clamping mechanism (101, 102) the respective second axis (A2, A2') upon deflecting into a predetermined deflected position corresponds to the respective first axis (A1, A1').

9. Medical device according to any of claims 1, 2 and 6 to 8, wherein for at least one clamping mechanism (101, 102) sidewalls (11a, 11a') of the respective first through-hole (11, 11'), sidewalls (13a, 13a') of the respective third through-hole (13, 13') and sidewalls (22a, 22a') of the respective second through-hole (22, 22') upon deflecting of the respective cantilever (20, 20') into a predetermined deflected position lie on the same cylindrical envelope, in particular on the same elliptical cylindrical envelope, in particular on the same circular cylindrical envelope.

10. Medical device according to any of claims 1 to 9, wherein for at least one clamping mechanism (101, 102) the respective cantilever (20, 20') in a resting position extends orthogonal to the respective first axis (A1, A1').

11. Medical device according to any of claims 1 to 10, wherein for at least one clamping mechanism (101, 102) the medical device body (10) traverse to the first axis (A1) has a respective slit (18, 18') extending from the respective space (17, 17') into the medical device body (10) for receiving a respective fix point side (28, 28') of the respective cantilever (20, 20'), wherein in particular the medical device body (10) traverse to the respective slit (18, 18') has at least one threaded through bore hole (18a, 18a') extending from an outer wall (10a) of the medical device body (10) to the respective slit (18, 18') for fixing the respective fix point side (28, 28') of the respective cantilever (20, 20') with a respective screw (18b, 18b').

12. Medical device according to any of claims 1 to 11, wherein for at least one clamping mechanism (101, 102) the respective space (17, 17') with respect to the respective first axis (A1, A1') opposite to the respective slit (18, 18') has a respective opening (19, 19') through which a respective free side (29, 29') of the respective cantilever (20, 20') extends over an outer wall (10a) of the medical device body (10), wherein the respective opening (19, 19') is dimensioned so as to allow bringing the respective cantilever (20, 20') from a resting position into a deflected position upon application of a force on the respective free side (29, 29') and traverse to the extension direction of the respective cantilever (20, 20').

13. Medical device according to claim 12, wherein the respective space (19, 19') through which a respective free side (29, 29') of the respective cantilever (20, 20) extends over an outer wall (10a) of the medical device body (10), has a stopper face (19a, 19a') being adapted for limiting a deflection and defining the deflected position, wherein in the respective defined deflected position sidewalls (11a, 11a') of the respective first through-hole (11, 11'), sidewalls (13a, 13') of the respective third through-hole (11, 11') and sidewalls (22a, 22a') of the respective second through-hole (22, 22') lie on the same cylindrical envelope.

14. Medical device according to any of claims 1 to 13, wherein for at least one clamping mechanism (101, 102) the respective cantilever (20, 20') at a respective free side (29, 29') of the respective cantilever (20, 20'), in particular at a respective free side (29, 29') of the respective cantilever (20, 20') extending over the outer wall (10a) of the medical device body (10) has a respective finger grip arrangement (27, 27').

15. Medical device according to any one of claims 2, 4 and 5 to 16, wherein the first axis (A1) and the further first axis (A1') are inclined with respect to each other.

16. Surgical targeting system having a medical device (100) according to any one of claims 1 to 15 as a targeting device and a secondary device (200) to be targeted, which secondary device has a shaft (210) which for at least one clamping mechanism (101, 102) is adapted to clearance fit the respective first through-hole (11, 11'), the respective second through-hole (22, 22') and the respective third through-hole (13, 13') when the respective cantilever (20, 20') is in a predetermined deflected position, and to be blocked in the respective first through-hole (11, 11'), the respective second through-hole (22, 22') and the respective third through-hole (13, 13') when the respective cantilever (20, 20') is in a deflected clamping position between the predetermined deflected position and the resting position so that the secondary device (200) is fixed in a targeted position of the secondary device (200) over the medical device (100).
